# EUROPEAN PATENT APPLICATION

(11) **EP 2 263 605 A1**
(43) Date of publication of application: **22.12.2010**
(21) Application number: 10009653.6
(22) Date of filing: 05.07.2007
(51) Int. Cl.: A61F 2/01, A61B 17/00, A61B 17/12, A61B 17/22

(54) **Device and method for preventing the undesired passage of emboli from a venous blood pool to an arterial blood pool**

(30) Priority: 20.11.2006 US 860393 P; 21.11.2006 US 866847 P
(62) Divisional of application: 07252700.5
(71) Applicant: SeptRx, Inc., Fremont, CA 94539 (US)
(72) Inventor: Greenhalgh, E. Skott, Wyndmoor Pennsylvania 19038 (US); Kleshinski, Stephen J., San Jose CA 95126 (US)
(74) Representative: Price, Nigel John King

(57) **Abstract**

The present invention relates generally to a device (10) and method for preventing the undesired passage of emboli from a venous blood pool to an arterial blood pool. The invention relates especially to a device (10) and method for treating certain cardiac defects, especially patent foramen ovales and other septal defects, through the use of an embolic filtering device (10) capable of instantaneously deterring the passage of emboli from the moment of implantation. The device consists of a frame (12), and a braided mesh (14) of sufficient dimensions to prevent passage of emboli through the mesh. The device (10) is preferably composed of shape memory allow, such as Nitinol, which conforms to the shape and dimension of the defect to be treated.

## Description

### CROSS-REFERENCE TO RELATED APPLICATIONS

This application claims the benefit of U.S. Provisional Application Nos. 60/860,393, filed 20 November 2006; and 60/866,847, filed 21 November 2006, which are incorporated by reference herein in their entireties.

### BACKGROUND OF THE INVENTION

### 1. Field of the Invention

The present invention relates generally to a device and method for preventing the undesired passage of emboli from a venous blood pool to an arterial blood pool. The invention relates especially to a device and method for treating certain cardiac defects, especially patent foramen ovales and other septal defects through the use of an embolic filtering device capable of instantaneously deterring the passage of emboli from the moment of implantation.

### 2. Description of Related Art

The fetal circulation is vastly different than the normal adult circulation. The blood circulating in a fetus is oxygenated by the placenta, not the developing lungs. Therefore, the fetal circulation directs only a small percentage of the circulating blood to the fetal lungs. Most of the circulating blood is shunted away from the lungs to the peripheral tissues through specialized vessels and foramens that are open ("patent" during fetal life. In most people these specialized structures quickly close after birth. Unfortunately, they sometimes fail to close and create hemodynamic problems that can be fatal if left untreated.

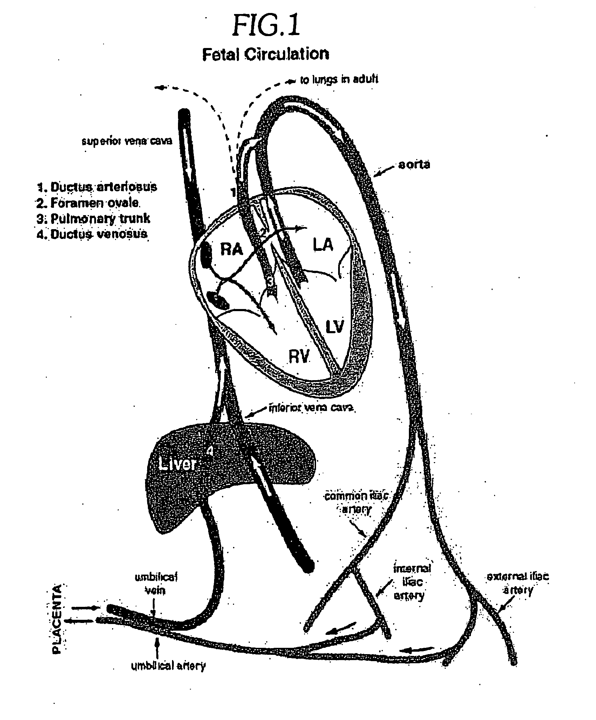

A diagram showing the blood circulation of a human fetus is illustrated in FIG. 1. The umbilical arteries branch off of the iliac arteries and deliver unoxygenated blood to the placenta. The fetal blood travels through the capillary bed in the placenta and transfers carbon dioxide to the maternal blood and takes oxygen and other nutrients from the maternal blood. The umbilical vein returns oxygenated blood to the fetus. Most of the oxygenated blood from the umbilical vein bypasses the developing liver and travels through a specialized vessel called the ductus venosus to the inferior vena cava and then into the right atrium. A good portion of the oxygenated blood from the inferior vena cava is directed across the right atrium and into the left atrium through a specialized curtain like opening in the heart called the foramen ovale. The blood from the left atrium then enters the left ventricle and then into the aorta where it travels to the head and other body tissues delivering the needed oxygen and nutrients.

The small amount of blood entering the right atrium that does not pass through the foramen ovale, most of which comes from the superior vena cava, flows into the right ventricle and then gets pumped into the pulmonary trunk and pulmonary arteries. Some of this blood is pumped into the developing lungs. However, the fetal lungs are collapsed which causes a high resistance to blood flow. Another specialized vessel, called the ductus arteriosus, is a vessel that connects the high pressure pulmonary artery to the lower pressure aorta. Therefore, most of the blood in the pulmonary artery flows into the lower pressure aorta through this specialized vessel.

Upon birth, the circulatory system goes through profound changes. The flow through the umbilical arteries and umbilical vein stops and consequently the flow through the musculature around the ductus venosus constricts and the blood flow through the ductus venosus stops. The lungs fill with air and the resistance to blood flow into the lungs drastically decreases. The corresponding pressure in the right atrium, right ventricle, and pulmonary arteries also decrease. The decrease in pressure in the right atrium causes the curtain like opening of the foramen ovale to close, driving more blood into the right ventricle and then to the lungs for oxygenation. Over time, the foramen ovale is replaced with a membrane called the fossa ovalis. Similarly, the decrease in pressure in the pulmonary arteries reduced the pulmonary arterial pressure to the same as or slightly less than the pressure in the aorta, which stops or reverses the flow through the ductus arteriosus. Once the muscular tissue of the ductus arteriosus is perfused with well oxygenated blood, the muscle begins to constrict and close the ductus arteriosus. The ductus arteriosus normally closes within about one week of life.

Usually over time, the unique openings of the fetal circulation become obliterated and a solid mass of tissue forms where these opening once were. However, in some people the opening remain. A patent ductus venosus after birth is very rare and almost always fatal. A patent ductus arteriosus occurs in about 1 out of every 5000 births. The patent ductus arteriosus once diagnosed is either medically treated or surgically ligated to close the ductus. In about one of four people, the foramen ovale does not seal shut, instead it remains patent. Such defects usually measure 10 mm or more in diameter and occupy one third or more of the length of the atrial septum in echocardiographic four chamber sections. Since the pressure in the left atrium is about two to four mm Hg greater than the pressure in the right atrium, the curtain like opening usually remains shut. However, if the pressure in the right atrium increases, such as upon heavy lifting or while performing a Valsalva type maneuver, the curtain like fold of tissue opens and the blood flows from the right atrium to the left atrium.

Studies have shown that adults with strokes of unknown origin, i.e., cryptogenic strokes, have about twice the normal rate of patent foramen ovales than the normal population. Although there is a correlation between strokes and patent foramen ovales, it is currently unknown why this correlation exists. It is theorized that blood clots and plaque that have formed in the peripheral venous circulation (in the legs for example) break off and travel to the heart. Normally, the clots and plaque get delivered to the lungs where it is trapped and usually cause no harm to the patient. Patients with a patent foramen ovale, however, have a potential opening that the clots or plaque can pass through the venous circulation and into the arterial circulation and then into the brain or other tissues to cause a thromboembolic event like a stroke. The clots may pass to the arterial side when there is an increase in the pressure in the right atrium. Then the clots travel through the left side of the heart, to the aorta, and then to the brain via the carotid arteries where they cause a stroke and the associated neurological deficits.

A number of atrial septal defects (ASD) closure devices have been developed and investigated in an attempt to develop a nonsurgical, transvenous method of occlusion of ASD. These include the Sideris Buttoned device, the Angel Wing Das device, the atrial septum defect occlusion system (ASDOS) device, the Amplatz Septal Occluder, the CardioSEAL/StarFlex devices, and the Gore/Helix devices. Unfortunately, each of these devices have distinct disadvantages and limitations ranging from the size of the device delivery sheath, ease of implantation, feasibility, safety and effectiveness. The Sideris buttoned device is made of a polyurethane foam occluder with a Teflon coated wire skeleton, which is positioned within the left atrium, and a polyurethane foam rhomboid shaped counteroccluder with a Teflon coated wire skeleton, which is positioned in the right atrium. The major disadvantage with this device is the lack of a centering mechanism. For this reason, use of the devices at least two times the size of the stretched ASD is required. (Sievert H. Koppeler P. Rux S: Percutaneous closure of 176 interarterial defects in adults with different occlusion devices--6 years of experience [abstract], J. Am. Coll. Cardiol 1999, 33:51 9A.) Consequently, closure of defects may become difficult because the required size may be too large for the atrial septum to accommodate, or the device may impinge critical structures. There are also reports that the retrieval of the Sideris button device after incorrect deployment is difficult. (See, e.g., Rigby, Michael L., The Era of Transcatheter Closure of Atrial Septal Defects, Heart; 81:227-228 (1999)).

The "Angel Wings" device comprises two square frames made of superelastic Nitinol wire, each square frame having four legs that are interconnected by flexible islets at the comers. The wire frames are covered by polyester fibers. There is a conjoint suture ring of the right and atrial discs, which allow self centering on deployment. The device is delivered through an 11-13 F Mullins sheath. The major disadvantage of using this device is the attendant risk of aortic perforation cause by its sharp eyelet comers. In fact, the Angel Wings device was withdrawn from further clinical trials because of this problem. (Syamaxundar Rao, P., M.D., Summary and Comparison of Atrial Septal Defect Closure Devices, Current Interventional Cardiology Reports 2000, 2:367-376 (2000)). The device is also ill-suited for treating fenestrated defects.

The atrial septal defect occlusion system (ASDOS) prosthesis (Microvena Corp., White Bear Lake, Minn.) consists of two umbrellas made of Nitinol and a patch of porous polyurethane attached to the left and right atrial devices. The device is introduced transvenously over a long veno-arterial guidewire and through an 11 F venous transeptal sheath. While the device is retrievable in the event of malpositioning before release of the device, it requires a complex procedure to implant, and the components are known to have a high incidences of thrombrosis. It is also reported that frame fractures have been detected in 20% of the patients treated with this device.

The Amplatzer device is the subject of U.S. Pat. No. 5,944,738 to Amplatzer, et al. This device is a saucer-shaped device formed from a mesh of fine Nitinol wires with a central connecting cylinder having a diameter similar to that of the stretched diameter of the defect. Thrombosis following implantation of the device is induced by three polyester patches. The device is delivered through a 6-10 F Mullins sheath. The primary disadvantage with this device is that it is ill-suited for closing fenestrated defects. Moreover, the device is a thick, bulky profile which dramatically increases the chances that the device will interfere with the heart's operation. Another disadvantage is its known capacity for incomplete endothelialisation with thrombus formation.

The CardioSEAL.RTM. device (NMT Medical is the subject of U.S. Pat. No. 6,206,907 to Marino, et al. This occlusion device is comprised of a center section to which stranded wire elastic shape memory fixation devices are attached. The fixation devices hold the occlusion devices in place once it is inserted into an aperture. Attached to the fixation devices are polyvinyl foam sheets which occlude the aperture. While the CardioSEAL is deemed to be relative easy to use, it is reported that, of all the devices, the CardioSEAL device has the highest incidence of arm fractures, which has raised serious issues concerning its safety. Moreover, the CardioSEAL device, like the Amplatzer device is relatively large, and requiring at least a 10 F or 11 F delivery systems, and an undue amount of hardware within the heart. These characteristics increase the chance that the device will interfere with the heart's operation, lend to residual shunting and/or embolization. The size of the CardioSEAL device also renders it less suitable for small children.

The STARflex.RTM. device (NMT Medical, Inc.) is an updated version of the CardioSEAL device, which includes a self-centering mechanism consisting of four flexible springs which pass between the two fabric disks. While this added feature may reduce the instances of residual shunting, the aforementioned defects and disadvantages of the CardioSEAL are still a concern.

In view of these drawbacks and related-risks, the method of choice to close a patent foramen ovale is still open heart surgery and ligation of the foramen ovale to close it. Surgery, however, is obviously associated with the usually risks of general anesthesia, open heart procedures, infections, etc. Thus, there is a need for a safe, cost-effective, and easily implantable device and method for preventing the passage of emboli from an arterial blood pool and a venous blood pool which is not subject to the defects and disadvantages of known devices.

### SUMMARY OF THE INVENTION

There is hereinafter described and illustrated an embolic filtering apparatus for treating septal defects, including patent foramen ovales. The embolic filtering device can have an embolic filter. The embolic filter can be made from metal, fiber, and/or polymer. The embolic filter can prevent the passage of emboli through the septal defect. The embolic filtering device can have a frame. The frame can allow the device to be secured within and or adjacent to the lumen of the septal defect.

The embolic filter is made by, for example, (1) swaging one end of a piece of tubular mesh at a first end with a first fastener (2) pulling the free end of the mesh over the first fastened end so that it overlaps the first portion; (3) swaging a second, center section of the tubular section to form a 3-dimensional ball-like structure having a first diameter portion with a second fastener; (4) extending the remaining free end of the tubular mesh back over the 3 dimensional ball-like structure of the first and second portions of the tubular mesh; and (4) swaging the free end of the tubular mesh with a third fastener to form an exterior 3-dimensional ball-like structure having a second diameter portion, within which the 3-dimensional ball-like structure of first diameter portion is disposed.

The mesh is removably is secured to at least one or more bases of the frame, and positioned between the arms thereof. The bases of the frame and the fasteners which secure the tubular mesh can be collars, for example, having central lumens. The aforementioned third-fastener is insertable into the lumen of at least one of the bases of the frame in order to secure the mesh to the frame. The lumens of the fasteners and bases are aligned along a common axis in order that a the embolic filtering device can be loaded onto a guide wire.

The frame can include at least one base and at least two arms which extend therefrom, between which the mesh is at least partially disposed. The frame can be made of metal, fabric and/or a polymer. The arms are positioned opposite one another and, in their resting state, are spaced apart from one another. When the device is composed of a shape memory metal, such as nitinol, the device can be collapsed into a catheter tube by compressing the arms of the frame toward one another, causing the length of the device to increase, and the width to decrease. As the device is released from the catheter tube, it reverts to its functional, relaxed state. The embolic filtering device may also be composed of non-shape memory metals, such as Elgiloy, cobalt chromium, and stainless steel, for example. Each arm includes at least one anchor positioned on the arms of the frames. The anchors can either be arcuate or linear in formation, depending on the shape of the patent foramen ovale to be treated, and are of sufficient rigidity to secure the device within the lumen of a septal defect.

To allow for non-invasive visualization of the device within a subject at least a portion of the frame or mesh is composed of or coated with a radiopaque material, such as tantalum. The device may also be treated with thrombin, collagen, hyluron, or a host growth factor to encourage and facilitate growth of tissue onto the device so as to further secure the device within the septal defect. The device can also be coated with an anticoagulant to deter formation of blood clots on the surface of the device.

In an exemplary embodiment, the mesh is composed of at least 96 strands of 0.002" diameter wire braided such that the wires are situated at an angle of 35.degree. relative to the longitudinal axis of the device. The interstices created by the braided wires are small enough such as to effectively filter emboli, thereby preventing emboli from passing through the patent foramen ovale, or other septal defect.

In another aspect of the invention, provided is a method of preventing the passage of emboli between a venous blood pool and an arterial blood pool by delivering the embolic filtering device to within, proximate to and/or adjacent to a passage between a venous blood pool and an arterial blood pool; and securing the device within, proximate to, and/or adjacent to said passage. The device can be delivered by a catheter to within and/or adjacent to the passage between the venous blood pool and the arterial blood pool.

### BRIEF DESCRIPTION OF THE DRAWINGS

Figure 1 is a schematic diagram of the fetal circulation;

Figure 2A illustrates a variation of the embolic filtering device;

Figure 2B illustrates a variation of the embolic filtering device;

Figure 2C illustrates a top view of the embolic filtering device illustrated in FIG. 2B;

Figure 2D illustrates a variation of the frame of the embolic filtering having two bases;

Figure 3 illustrates a variation of the embolic filtering device with a frame having one base;

Figure 4 illustrates a variation of the embolic filtering device and delivery mechanism;

Figure 5A illustrates a variation of the preferred embolic filtering device;

Figure 5B and 5C illustrate a variation of the embolic filter device within a patent foramen ovale;

Figures 6A and 6B illustrate a variation of the embolic filter device; and

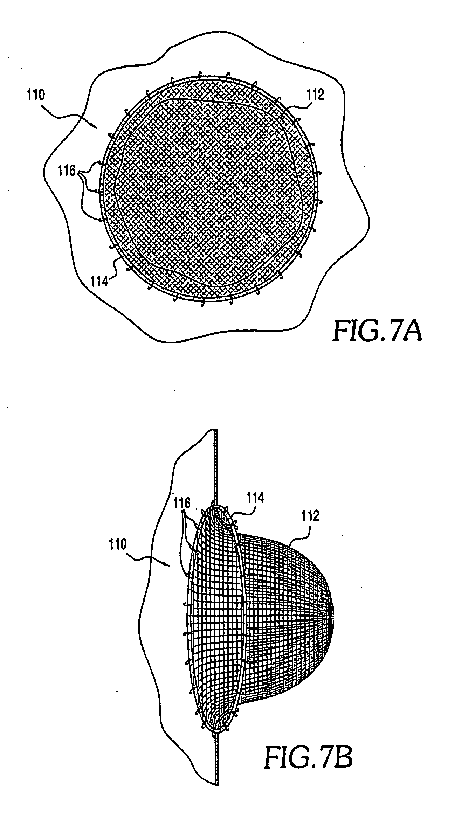

Figures 7A and 7B illustrated a variation of the embolic filter device.

Figures 8a and 8b illustrate various sections of tissue having a tunnel defect.

Figure 9 illustrates the tunnel defect of Figure 8a or 8b.

Figure 10 illustrates a variation of a method of deploying a variation of the embolic filtering in a tunnel defect.

### DETAILED DESCRIPTION OF THE INVENTION

Disclosed are methods and apparatuses for preventing the passage of emboli between a venous blood pool and an arterial blood pools using devices for creating a barrier to the conducting of emboli at a passage between a venous blood pool and an arterial blood pool. The device can treat cardiac defects, such as patent foramen ovale or other atrium septal defects. Although referred to as a filtering device, the device can work by any mechanism including or not including filtering. For example, the embolic filtering device can act as a scaffold for tissue to grow.

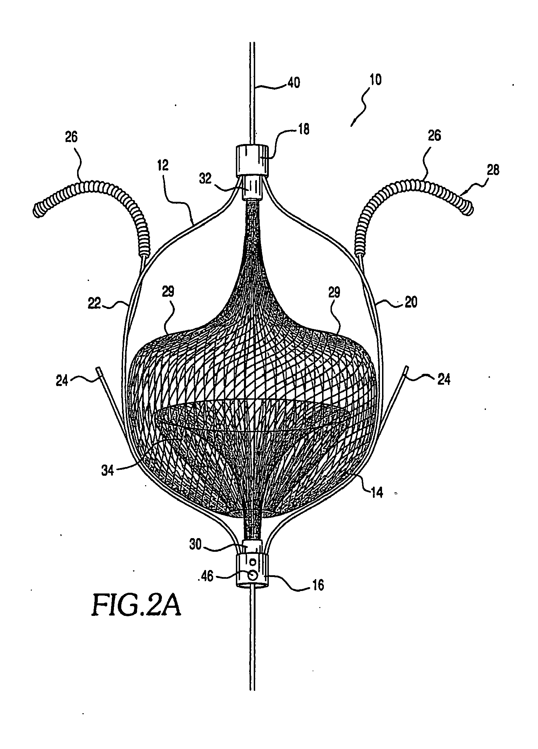

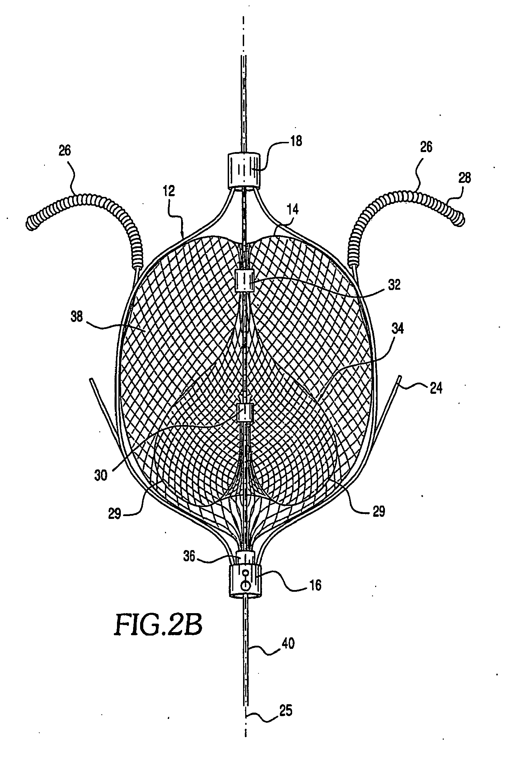

FIG. 2A illustrates an embolic filtering device 10 comprising a frame 12 and an embolic filter 14 comprising a mesh of stranded fabric, wire, or combination thereof. Any and/or all elements of the embolic filtering device 10, including the frame 12 and the embolic filter 14, can be entirely or partially biodegradable and/or bio-inert (e.g., non-biodegrading). After being deployed in the patient, the embolic filtering device can completely or partially biodegrade. For example, the embolic filtering device 10 can be made in-part from a first metal that is biodegradable and/or in-part from a second metal that is non-biodegradable, and partially from a first polymer that is biodegradable, and partially from a second polymer that is non-biodegradable. For example, the embolic filter 14 can be biodegradable and the frame 12 can be non-biodegradable. Also for example, the embolic filter 14 can be non-biodegradable and the frame 10 can be biodegradable.

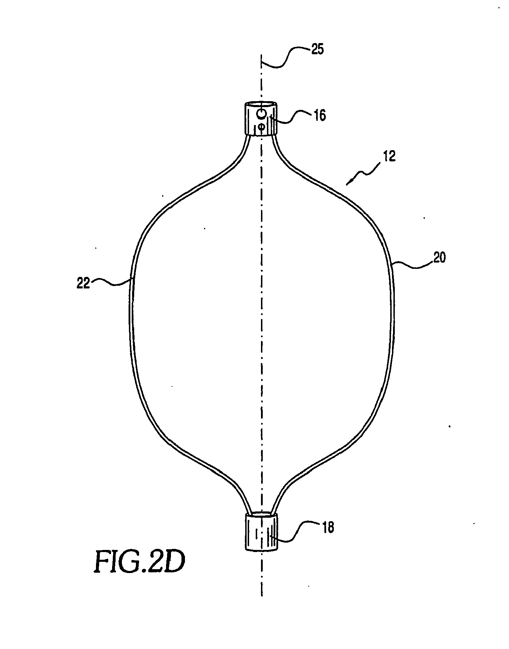

FIG. 2D illustrates one frame 12 without embolic filter 14 attached. Frame 12 can have a first base 16 and a second base 18. Each end of arms 20 and 22 can be connected to first base 16 and second base 18, such that the lumens of first base 16 and second base 18 are in line with longitudinal axis 24 of frame 12. Arms 20 and 22 are preferably formed of a shape memory metal, e.g., Nitinol, and formed such that, in the resting state, they are spaced apart from one another.

Referring to FIG. 2A, right anchors 24 can extend laterally from each of arms 20 and 22 proximate to first base 16. Right anchors 24 can be of any shape or formation suitable for delivering embolic filtering device 10 to the desired location and securing it in place. In a preferred embodiment, right anchors 24 are preferably linear or arcuate, and extend outward from frame 12 and away from first base 16, in the direction of second base 18, at an acute angle relative to longitudinal axis 25. The desired length of right anchors 24 and the position from which they extend from arms 20 and 22 will depend primarily on the size of the passage or defect to be treated. In any event, the right anchors 24 are of sufficient length to securely engage tissue within and/or adjacent to the septal defect. For example, when treating a patent foramen ovale, right anchors 26 preferably engage tissue within and/or adjacent to the right-atrial opening of the patent foramen ovale. Extending arcuately and/or laterally from the portion of arms 20 and 22 proximate second base 18 are left anchors 26. Left anchors 26 can be of any shape or formation suitable for delivering embolic filtering device 10 to the desired location and securing it in place; however, it has been found that arcuate or coiled anchors are most suitable for effectively securing the device within the area of interest. As with right anchors 24, left anchors 26 are of sufficient length to securely engage tissue within and/or adjacent to the septal defect to be treated. For example, when treating a patent foramen ovale, left anchors 26 preferably engage tissue within and/or adjacent to the left-atrial opening patent foramen ovale. In a preferred embodiment, right anchor 24 and left anchor 26 are covered with tantalum coil 28, or other radiopaque material, to allow for visualization of the position and location of embolic filtering device 10 after implantation in a subject. First base 16 and second base 18 and, for that matter, any portion of device 10 can likewise be compromised of radiopaque materials to provide even more visual points of reference in the imagery of embolic filtering device 10.

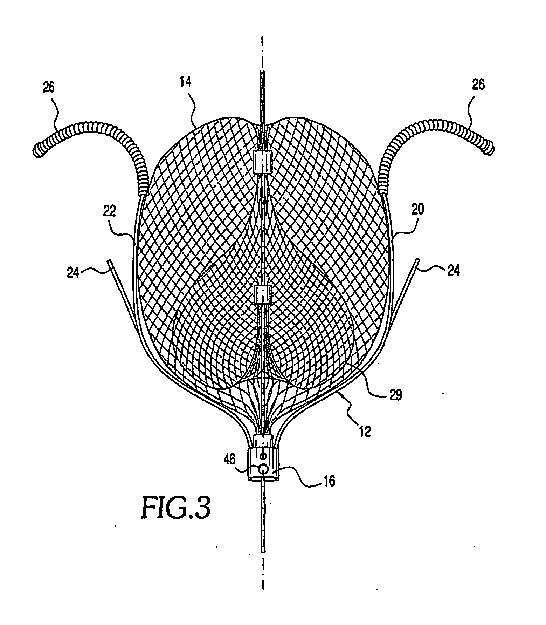

FIG. 3 illustrates a frame 12 having first base 16, but without second base 18, and shortened arms 20 and 22. By eliminating second base 18, the amount of hardware implanted in the passage to be treated is minimized. Since, as discussed below, second base 18 resides closest to the left atrium of the heart when embolic filtering device 10 is used to treat a patent foramen ovale, eliminating second base 18 minimizes the amount of hardware adjacent to or within the left atrium, decreasing the chance the operation of the left atrium will be comprised, and reducing the surface area upon which blood clots can form.

Embolic filter 14 can be fixedly or removably attached or coupled to frame 12. Embolic filter 12 can have a plurality of braided wire strands having a predetermined relative orientation and interstitial space between the strands. The number and diameter of the wires used can be selected to achieve the desired density and stiffness of the fabric, and the known size of the emboli sought to be filtered. The wire mesh can have at least 96 strands of 0.002" diameter wire, situated at an angle of approximate 35° relative to the longitudinal axis 24. Wire strand materials can be a cobalt-based low thermal expansion alloy (e.g., Elgiloy), nickel-based high temperature high-strength "superalloys" (e.g., Nitinol), nickel-based treatable alloys, a number of different grades of stainless steel, and polymers, including polyester, nylon, polytetrafluoroethylene (PTFE), polyurethane, polyaryletheretherketone (PEEK), and polyglycolic acid (PGA), polylactide (PLA), polyepsilon-caprolactone, polyethylacrylate (PEA), or combinations thereof. Platinum and alloys of platinum can also be co-braided, co-knitted or co-woven into mesh 14 to assist in determining where mesh is positioned within the patent foramen ovale. The wire strands can be made from a shape memory alloy, NiTi (known as Nitinol) which is an approximately stoichiometric alloy of nickel and titanium and may also include minor amounts of other metals to achieve desired properties. The frame 12 of device 10, and its components, including base 16, base 18, right arms 24 and left arms 26, can be made from shape memory alloys. Such alloys tend to have a temperature induced phase change which will cause the material to have a preferred configuration which can be fixed by heating the material above a certain transition temperature to induce a phase change in the material. When the alloy is cooled, the alloy can "remember" the shape it was in during the heat treatment and will tend to assume that configuration, unless constrained from doing so.

Handling requirements and variations of NiTi alloy compositions are known in the art. For example, U.S. Pat. No. 5,067,489 (Lind) and U.S. Pat. No. 4,991,602 (Amplatz et al.), the entire teachings of which are herein incorporated by reference, discuss the use of shape memory NiTi alloys in guide wires. NiTi alloys can be very elastic (e.g., "superelastic" or "pseudoelastic"). This elasticity allows device 10 to return to a preset configuration after deployment from a catheter or other delivery device. The relaxed configuration can be defined by the shape of the fabric when it is deformed to generally conform to the molding surface of the mold in which it was created. The wire stands are manufactured by standard braiding processes and equipment.

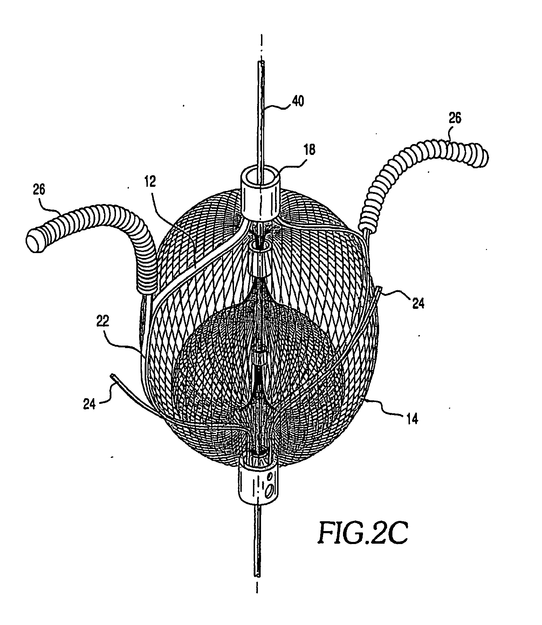

Embolic filter 14 can be in the shape of a three-dimensional ball or sphere, as exemplified in FIGS. 2A and 2C. Starting with a tubular piece of braided mesh or the like, the three-dimensional ball or sphere, as exemplified in FIG. 2A, is, for example, made by swaging a first end of the mesh with a first fastener 30, and pushing said first fastener 30 upwards into the lumen of the tubular mesh, to create interior lobes 29. A center portion of the mesh is then swaged with a second fastener 32, creating an interior embolic filter portion 34. The remaining mesh is then extended back over said first fastener 30 and interior embolic filter portion 34, and the second end of the braided tubular mesh is swaged with a third fastener 36. First fastener 30, second fastener 32, and interior embolic filter portion 34 are in effect situated within exterior embolic filter portion 38. Third fastener 36 is situated outside of said exterior embolic portion 38. In a preferred embodiment, fasteners 30, 32 and 36 are collars having a central lumen. The lumens of the collars are substantially aligned along a common longitudinal axis 25, and dimensioned to receive a guide wire 40. Embolic filter 14 is preferably secured to frame 12 by inserting third fastener 36 into the lumen of first base 16 of frame 12. To reduce the chance of third fastener 36 from disengaging from first base 16, third fastener 36 and first base 16 can be coupled together, either by a mechanical locking means such as that created by a press fit, a melted polymer interlock, or hot melt adhesive, or by plasma welding. Plasma welding is the preferred coupling method, as it allows first base 16 to be shorter, since no portal is required on the base. When coupled to frame 12, embolic filter 14 resides at least partially between arms 20 and 22, such that the lumens of fasteners 30, 32, and 36 are substantially aligned with the lumens of first base 16 and second base 18 (if employing a frame with second base 18), along longitudinal axis 24. A plug composed of collagen, fabric, an adhesive, polymer or foam, for example, may be disposed within the aforementioned sphere to further deter the passage of embolic through the mesh.

FIG. 2A illustrates an embolic filter 14 that can have a first end comprising at least one lobe-like formation and a second end which tapers inward therefrom.. To make this embodiment, a piece of tubular mesh of suitable length, for example, is swaged at a first end by a first fastener 30. This first fastened end is then pushed into the lumen of the tubular mesh to form lobes 29. The second end of the mesh is then swaged by a second fastener 32. This embodiment is attached to frame 12 by securing first fastener in the lumen of base 16, and securing second fastener 32 in the lumen of base 18. As discussed above, fasteners 30 and 32 are collars having central lumens. The lumens of the collars are substantially aligned along a common longitudinal axis, and dimensioned to receive a guide wire 40.

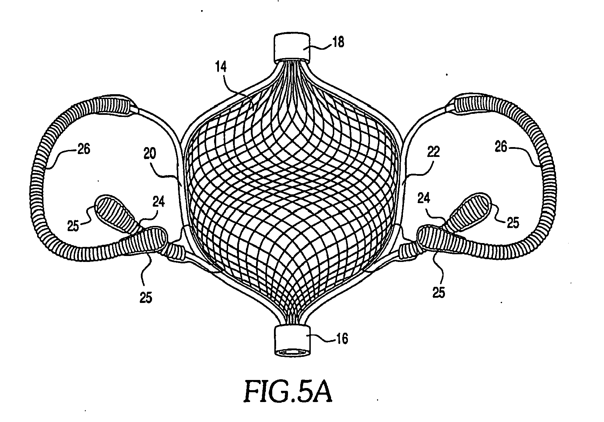

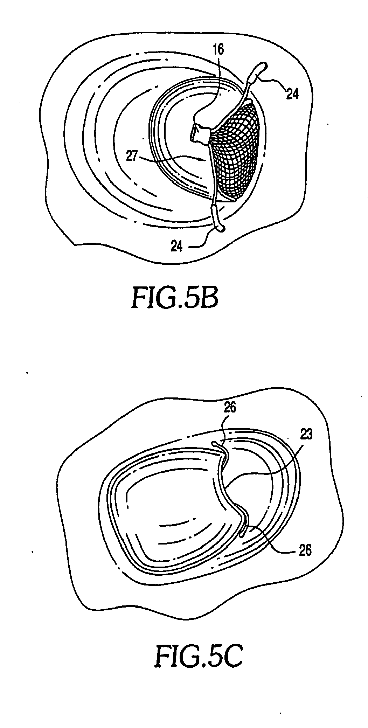

FIG. 5A, illustrates an embolic filtering device 10 having right anchors 24 which are specifically designed to engage the perimeter of the tissue defining the right-atrial opening 23 of the patent foramen ovale, as illustrated in FIG. 5B. The ends of right anchors 24 of this embodiment can reside against or adjacent to the outside of the tissue wall defining the patent foramen ovale. Right anchors 24 can be slightly longer dimension and at least slightly arcuate in shape to facilitate this methodology. The ends of right anchors 24 can have or include protective caps 27 at their distal ends. Caps 25 can be composed of rubber, plastic, or any other suitable material for covering the ends of anchors 27, and may also comprise radiopaque materials, for example, in order to allow post-implant visualization of the location and positioning of anchors 24 after implant.

Mesh 14 can be manufactured in a variety of ways. For example, mesh 14 does not necessarily need to be spherical, or have both an interior and exterior embolic portion, as discussed above. Mesh 14 can be of any shape and dimension suitable to deter the passage of embolic material between a venous blood pool and an arterial blood pool, and can include any number of layers. The interstices between the strands forming mesh 14 can be of sufficient area to filter emboli.

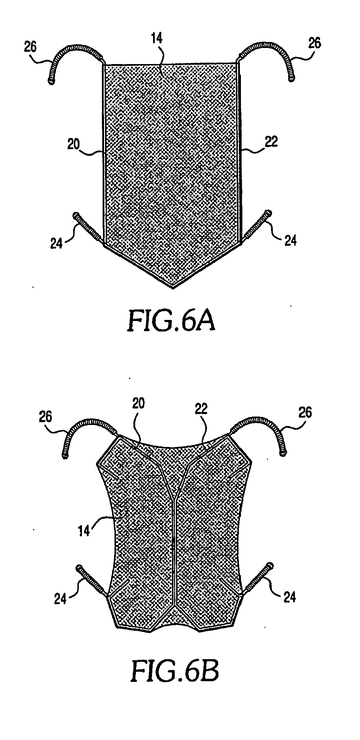

The design and dimensions of frame 12 can also be manufactured in a variety of ways. FIGS. 6A and 6b illustrate that arms 20 and 22 can be effectively decoupled from one another, such that the tissue distension function of embolic filtering device 10 is provided separately by each individual legs of the device. This allows embolic filtering device 10 to be more compact, and to better fill gaps and meet the contours of the patent foramen ovale. Particularly with respect to the embodiments shown in FIG. 6A and 6B, should be recognized that the size of mesh 14 need not be large, but can cover only arms 20 and 22 and still be effective in treating patent foramen ovales.

Device 10 provides distinct advantages and improvements over known patent-foramen ovale-treatment devices. First, the elasticity and ball-like structure of mesh 14, enables device 10 to treat a patent foramen ovales, or other septal defects, of any shape and dimension with equal effectiveness. This is because mesh 14 is compressible along its entire length. Thus, it does not matter if the patent foramen ovale is fenestrated, as the elasticity of mesh 10 will allow it to conform to the substantially exact shape and dimension of the patent foramen ovale. Mesh 14 can also be annealed to have a 3-dimensional to help fill any gaps within the patent foramen ovale space. Thus, the post-implant leakage along the perimeter of known devices caused by their inability to accommodate irregular shaped defects is eliminated. Second, device 10 has substantially less surface compared to known devices, thereby reducing the risk of dangerous blood clot formation on the exterior of the device. Third, contrary to known devices which do not prevent passage of emboli through the defect until tissue growth onto the device occludes the defect, the interstices between the stands of braided mesh 14 of the present invention are small enough to effectively filter emboli as soon as device 10 is implanted. Thus, device 10 offers immediate protection against the passage of emboli at the moment of implant.

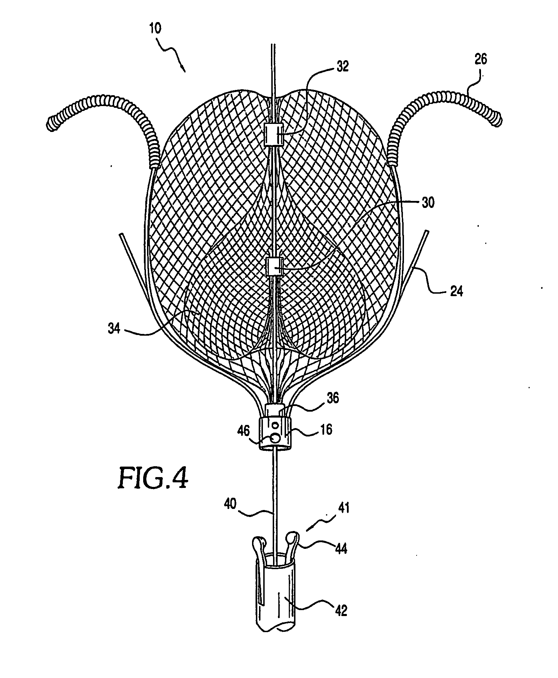

The embolic filtering device 10 can prevent the passage of emboli between a venous blood pool and an arterial blood pool. For purposes of exemplary illustration, the method of the invention is herein exemplified through discussion of a method of treating a patent foramen ovale (PFO). However, the embolic filtering device can be used to prevent the passage of emboli between any septal defect and/or arterial venous blood pool and arterial blood pool. To deliver the embolic filtering device 10 of the patent foramen ovale, embolic filtering device 10 is loaded into a delivery system 41 comprising a catheter 42, exemplified in FIG. 4. The embolic filtering device 10 can be loaded onto a guide wire 40 by inserting the guide wire through the lumens of first base 16, the lumens of fasteners 30, 32, and 36, if employing a frame 12 with second base 18, the lumen of second base 18. A pair of forceps 44, as exemplified in FIG. 4, or other grasping device, is used to grasp embolic filtering device 10. First base 16 can have a recess 46 for receiving forceps 44, such that forceps 44 are positioned within recess 46 to more securely grasp embolic filtering device 10, and to deter embolic filtering device 10 from detaching from forceps 44. With embolic filtering device 10 secured by forceps 44 embolic filtering device 10 is pulled into catheter 42. As embolic filtering device 10 is pulled into catheter 42, the force of the catheter walls against first base 16 of frame 12 will force side walls 20 and 22, and left anchors 24 and right anchors 26 inward toward one another. Embolic filtering device 10 will gradually collapse as it is pulled into catheter 42.

Using catheter 42, embolic filtering device 10 is delivered to the patent foramen ovale, or other passage between a venous blood pool or arterial blood pool, to be treated. In particular, the distal end of catheter 42 is extended through the patent foramen ovale from the right atrial side to the left atrial side. With the distal end of catheter 40 positioned in the left atrium adjacent to the patent foramen ovale, forceps 44 are used to withdraw embolic filtering device 10 from catheter 42. As embolic filtering device 10 is withdrawn, embolic filtering device 10 will gradually expand from its collapsed position and into its memorized shape and/or in conformance to the shape and dimension of the patent foramen ovale being treated. With the distal end of catheter 42 positioned in the left atrium, adjacent to the patent foramen ovale, embolic filtering device 10 is withdrawn from catheter 42, while catheter 42 is slowly pulled back through the patent foramen ovale in the direction of the right atrium. Left anchors 26 can be withdrawn first. As catheter 42 is pulled back, left anchors 26 can securely engage the walls defining the patent foramen ovale, for example, the tissue defining the perimeter of the left-atrial opening 23 of the patent foramen ovale, as shown in FIG. 5C. As catheter 42 is pulled back further, the engagement of left anchors 26 onto the tissue defining the perimeter of the left-atrial opening 23 of arms 20 and 22 will prevent embolic filter device 10 from being pulled through the patent foramen ovale, and embolic filter 14 can emerge within the patent foramen ovale, and can gradually expand apart from one another in returning to the shape memorized orientation. As arms 20 and 22 expand apart from one another, pressure will be exerted onto the tissue defining the lumen of the patent foramen ovale, thereby acting as a tissue distension device. The tissue defining the patent foramen ovale will naturally press inward against mesh 14, in effect squeezing the device within the patent foramen ovale. As catheter 42 is pulled back yet further, right anchors 24 will emerge and, as they expand to their memorized shape, will also forcibly engage, for example, the walls defining the patent foramen ovale, or the perimeter of the tissue defining right atrial opening 27 of the patent foramen ovale. If using the embolic filter device illustrated in FIG. 5A, for example, right anchors 24 will engage the tissue defining the outside perimeter defining the right-atrial opening 27 of the patent-foramen ovale, as illustrated in FIG. 5B. In its memorized shape, embolic filter 14 should be sized to engage the walls defining the patent foramen ovale with sufficient force to prevent emboli from passing between the exterior of the embolic filter 14 and the walls of defining the patent foramen ovale. Further, the force created from blood flowing from the right atrium to the left atrium against right anchors 24 facilitates the securing of right anchors 24, and helps prevent embolic filtering device 10 from becoming dislodged from its intended position.

The device can be secured in place by adhesives, sutures, hooks, barbs, or other such means. To enhance recovery subsequent to implanting embolic filtering device 10 frame 12 and/or mesh 14 can be coated with known drugs suitable for that purpose. Non-pharmacological methods can also be used to promote healing, including ultrasound, radiofrequency, radiation, mechanical vibration, other non-pharmacological healing method, or combinations thereof.

Prior to disengaging embolic filtering device 10 from forceps 44 and removing catheter 42 from the subject, known radiological techniques can be employed to insure that embolic filtering device 10 is properly positioned and secured within the patent foramen ovale. If the position of embolic filtering device 10 needs to be altered, forceps 44, while still secured to embolic filtering device 10, can be used to reposition embolic filtering device 10; otherwise, forceps 44 are disengaged from embolic filtering device 10, and forceps 44, catheter 42, and guide wire 40 are withdrawn. Should embolic filter device 10 later become disengaged, disoriented, damaged or otherwise need to be removed, forceps 44 can be used to easily reposition or recover embolic filter device 10, as necessary. To facilitate the ease by which embolic filter device 10 is repositioned or recovered, base 16 can be coated with a suitable material to deter tissue from covering recess 46.

From the moment that embolic filtering device 10 is inserted, emboli are effectively filtered by embolic filtering device 10. Since blood travels from the direction of the right atrium to the left atrium, the portion of embolic filter 14 having a higher density of mesh, e.g., lobes 29 and/or interior embolic filter portion 34, are positioned on the right atria side to decrease the chances that emboli will penetrate into the left atrium. The design of embolic filtering device 10, however, is such that if emboli pass through the right side of embolic filter 14, there is still a significant chance that the portion of embolic filter 14 positioned on the left atrial side will prevent the emboli from passing into the left atrium.

Thus, unlike known devices for treating patent foramen ovale or atrial septal defects, for example, it is not necessary for thrombi to collect on the embolic filtering device 10 before the passage of emboli are effectively deterred. However, if total occlusion of the passage is desired, embolic filtering device 10 the embolic filter 14 can be treated with materials to promote thrombrosis, tissue in-growth, or adhesions. Embolic filter 14 can also be treated with anticoagulants to discourage blood clot formation on the device 10.

The primary function of frame 12 is to facilitate the delivery, positioning and securing of the embolic filter 14 within and/or adjacent to a passage between a venous blood pool and an arterial blood pool. It should be appreciated, however, that embolic filter 14 can be employed by itself, without frame 12, by securing embolic filter 14 by other means, e.g. sutures, hooks, etc., to deter the passage of emboli through a passage between a venous blood pool and an arterial blood pool. Further, embolic filter 14 can be of virtually any shape, spherical, round, oval or flat, so long as it retains its ability to filter emboli.

In another aspect of the invention, as exemplified in FIGS. 6A and 6B, provided is an embolic filter device 100 composed of a mesh 112 and a frame 114, to which mesh 112 is attached. Mesh 112 can be composed of any suitable material, including fabric, metal (e.g. shape memory metal or non-shape memory metal), or polymer, and can be of any shape (e.g., round, oval, or flat) or size suitable for the opening to be treated. Frame 114 can also be composed of any suitable material. For example, frame 114 can be composed of fabric, if rigidity is not required to support the opening to be treated. Alternatively, frame 114 can be composed of plastic, metal or the like, so as to act as a stent to give support to the orifice through which the passage of embolic is to be deterred. Depending on the particular use, mesh 112 and/or frame 114 can be absorbable or non-absorbable. To deter the passage of emboli from a passage between a venous blood pool and an arterial blood pool, embolic filtering device 110 can block the passage between a venous blood pool and an arterial blood pool. Using the example of a patent foramen ovale, embolic filtering device 100 can be attached to tissue adjacent to the patent foramen ovale by for example, sutures, barbs, hooks, glue, or any other suitable attaching means 116 to, in effect, create a screen covering the right atrial and/or left atrial openings, and/or within the lumen of the patent foramen ovale. The attaching means 116 can be on frame 114. The attaching means 116 can be placed at any suitable location on embolic filter device 100. Once in place, embolic filtering device 110 effectively deters the passage of emboli from the right atrium to the left atrium via the patent foramen ovale. Embolic filter device may be delivered either percutaneously, surgically, or via a catheter, depending on the area to be treated.

The frame 12 can be made from a biodegradable and a non-biodegradable polymer. The frame 12 can be made from a polymer and/or a metal. For example the frame 12 can be made from a biodegradable, a non-biodegradable polymer and a metal.

The embolic filter 14 can be made from a non-woven material. For example, the embolic filter 14 can be made from felt, paper, scrim cloth, a melted material, a blown material, film (e.g., textured film, slit film), a single layer of material, multiple layers of material, individual filaments, individual yarns, individual threads, random fibrils, gels, swelling polymers, foams, textured threads (e.g., hairy, bulky, tangled bundles), coils (e.g., 3-dimensional coil shapes), or combinations thereof.

The embolic filter 14 can be made from biodegradable polymer thread and/or non-biodegradable polymer thread. The embolic filter 14 can be made from thread that is made from mixed biodegradable and non-biodegradable polymer. The embolic filter 14 can be made from polymer threads and/or metal threads. For example, the embolic filter 14 can be made from Nitinol thread mixed with PET and/or PGA thread. The embolic filter 14 can be made from thread that is made from mixed polymer (i.e., biodegradable and/or non-biodegradable) and metal. For example, the embolic filter 14 can be made from thread made from Nitinol mixed with PET and/or PGA.

The embolic filter device 10 can be configured to stop motion (i.e., anchoring), after deployment, of the embolic filter device 10 within the biological tunnel to which embolic filter device 10 is deployed. The anchoring can stop migration of the embolic filtering device 10.

Friction can anchor the embolic filtering device 10. Tissue of the biological tunnel can bind to the frame 12. The binding can be accomplished by ingrowth of the tissue into or around the frame 12. The binding can be accomplished by surface friction (e.g., static and/or dynamic) between the frame 12 and the tissue. Tissue of the biological tunnel can bind to the embolic filter 14 (i.e., shroud). The binding can be accomplished by ingrowth of the tissue into or around the embolic filter 14. The binding can be accomplished by surface friction (e.g., static and/or dynamic) between the embolic filter 14 and the tissue. All or part of the surfaces of the embolic filter device 10, such as the frame 12 and/or the embolic filter 14, can be increased with surface textures (e.g., knurling, pebbling, ridging, roping, or combinations thereof), encrusting (e.g., with granular materials, such as diamond, sand, the material of the surface of the embolic filter device 10, any other material listed herein, or combinations thereof), increased radial or planar forces (e.g., squeezing the septal tissue between arms of the embolic filter device 10), vacuum (e.g., by an active vacuum, or active or passive suction cups, such as micro suction cups), 3-dimensional shapes such as coils used to help grab the tissue, or combinations thereof.

The embolic filter device 10, for example on the frame 12 and/or embolic filter 14, can have a bioadhesive. The bioadhesive can be a glue or a drug. The bioadhesive can be configured to attach to the tissue. The embolic filter device 10 can be adhered or otherwise bonded to the tissue by application of heat, RF energy, ultrasound energy, magnetic resonance (e.g., MRI), x-ray radiation, or combinations thereof.

The embolic filter device 10 can have one or more anchors. The anchor can be an active anchor. The active anchor can move actively (e.g., a spring-loaded barb) when deployed. The active anchor can pierce tissue with or without barbs when the embolic filter device 10 is deployed.

The anchor can be a passive anchor. The passive anchor can be a loop, hook, tooth, tab, finger of material used to grab or loop over tissue or work into nooks and crannies within tunnels, or combinations thereof.

The embolic filter device 10 can be manufactured from a round tube or flat sheet of material. The embolic filter device 10 can be made by laser cutting, weaving, stamping, die-cutting, molding, or made in any combination of methods thereof.

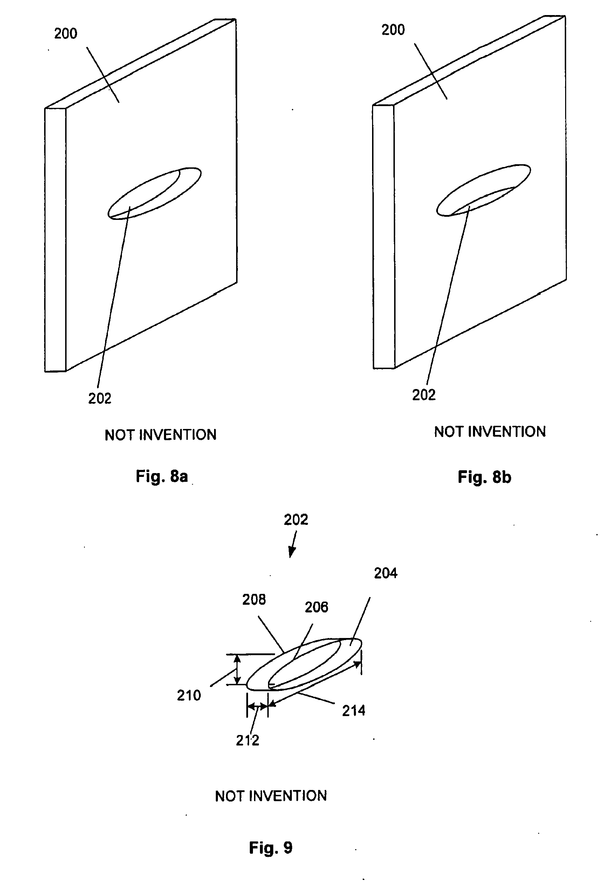

Figure 8a illustrates a section of tissue 200 that can have a tunnel defect 202 passing through the tissue 200. The tunnel defect 202 can be substantially perpendicular to the face of the tissue 200. For example, the tunnel defect 202 can be an atrial septal defect (ASD). Figure 8b illustrates that the tunnel defect 202 can be at a steep angle or substantially parallel to the face of the tissue 200. For example, the tunnel defect 202 can be a patent foramen ovale (PFO).

Figure 9 illustrates that the tunnel defect 202 can have a defect front face 204 and a defect back face (not shown). A defect front lip 206 can be defined by the perimeter of the defect front face 204. A defect back lip 208 can be defined by the perimeter of the defect back face. The tunnel defect 202 can have a defect height 210, a defect depth 212 and a defect width 214.

The embolic filtering device 10 can be used to treat any tunnel defect.

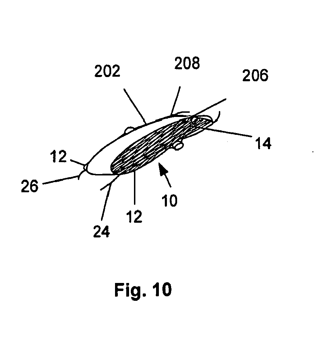

Figure 10 illustrates that the embolic filtering device 10 can be deployed in the tunnel defect 202. After deployment, the embolic filtering device 10 can be located entirely, substantially, or partially within the tunnel defect 202. The frame 12 can be in substantial contact with wall of the tunnel defect 202. The embolic filter 14 can be in substantial contact with wall of the tunnel defect 202.

The embolic filtering device 10 can stop blood flow through the tunnel defect 202 quickly or slowly (i.e., time effect). The embolic filtering device 10 can partially, substantially or completely impede or stop fluid (e.g., blood) and solid (e.g., blood clot) flow through the tunnel defect 202 at the time of deployment. The embolic filtering device 10 can partially, substantially or gradually increasingly impede or stop fluid (e.g., blood) and solid (e.g., blood clot) flow through the tunnel defect 202 as time progresses after deployment. The tissue 200 around the tunnel defect 202 can grow or otherwise heal onto the embolic filtering device 10, for example onto the frame 12 and/or the embolic filter 14. The tissue grown or healed onto the embolic filtering device 10 can further impede or stop fluid (e.g., blood) and solid (e.g., clot) flow through the tunnel defect 202.

The embolic filtering device 10, for example the frame 12 and/or embolic filter 14, can plug the tunnel defect 202.

Any or all elements of the embolic filtering device and/or other devices or apparatuses described herein can be made from, for example, a single or multiple stainless steel alloys, nickel titanium alloys (e.g., Nitinol), cobalt-chrome alloys (e.g., ELGILOY® from Elgin Specialty Metals, Elgin, IL; CONICHROME® from Carpenter Metals Corp., Wyomissing, PA), nickel-cobalt alloys (e.g., MP35N® from Magellan Industrial Trading Company, Inc., Westport, CT), molybdenum alloys (e.g., molybdenum TZM alloy, for example as disclosed in International Pub. No. WO 03/082363 A2, published 9 October 2003, which is herein incorporated by reference in its entirety), tungsten-rhenium alloys, for example, as disclosed in International Pub. No. WO 03/082363, polymers such as polyethylene teraphathalate (PET), polyester (e.g., DACRON® from E. I. Du Pont de Nemours and Company, Wilmington, DE), polypropylene, aromatic polyesters, such as liquid crystal polymers (e.g., Vectran, from Kuraray Co., Ltd., Tokyo, Japan), ultra high molecular weight polyethylene (i.e., extended chain, high-modulus or high-performance polyethylene) fiber and/or yam (e.g., SPECTRA® Fiber and SPECTRA® Guard, from Honeywell International, Inc., Morris Township, NJ, or DYNEEMA® from Royal DSM N.V., Heerlen, the Netherlands), polytetrafluoroethylene (PTFE), expanded PTFE (ePTFE), polyether ketone (PEK), polyether ether ketone (PEEK), poly ether ketone ketone (PEKK) (also poly aryl ether ketone ketone), nylon, polyether-block co-polyamide polymers (e.g., PEBAX® from ATOFINA, Paris, France), aliphatic polyether polyurethanes (e.g., TECOFLEX® from Thermedics Polymer Products, Wilmington, MA), polyvinyl chloride (PVC), polyurethane, thermoplastic, fluorinated ethylene propylene (FEP), absorbable or resorbable polymers such as polyglycolic acid (PGA), poly-L-glycolic acid (PLGA), polylactic acid (PLA), poly-L-lactic acid (PLLA), polycaprolactone (PCL), polyethyl acrylate (PEA), poly ester amide (PEA), polydioxanone (PDS), and pseudo-polyamino tyrosine-based acids, extruded collagen, silicone, zinc, echogenic, radioactive, radiopaque materials, a biomaterial (e.g., cadaver tissue, collagen, allograft, autograft, xenograft, bone cement, morselized bone, osteogenic powder, beads of bone) any of the other materials listed herein or combinations thereof. Examples of radiopaque materials are barium sulfate, zinc oxide, titanium, stainless steel, nickel-titanium alloys, tantalum and gold.

Any or all elements of the embolic filtering device and/or other devices or apparatuses described herein, can be, have, and/or be completely or partially coated with agents and/or a matrix a matrix for cell ingrowth or used with a fabric, for example a covering (not shown) that acts as a matrix for cell ingrowth. The matrix and/or fabric can be, for example, polyester (e.g., DACRON® from E. I. Du Pont de Nemours and Company, Wilmington, DE), polypropylene, PTFE, ePTFE, nylon, extruded collagen, silicone or combinations thereof.

The embolic filtering device and/or elements of the embolic filtering device and/or other devices or apparatuses described herein and/or the fabric can be filled, coated, layered and/or otherwise made with and/or from cements, fillers, glues, and/or an agent delivery matrix known to one having ordinary skill in the art and/or a therapeutic and/or diagnostic agent. Any of these cements and/or fillers and/or glues can be osteogenic and osteoinductive growth factors.

Examples of such cements and/or fillers includes bone chips, demineralized bone matrix (DBM), calcium sulfate, coralline hydroxyapatite, biocoral, tricalcium phosphate, calcium phosphate, polymethyl methacrylate (PMMA), biodegradable ceramics, bioactive glasses, hyaluronic acid, lactoferrin, bone morphogenic proteins (BMPs) such as recombinant human bone morphogenetic proteins (rhBMPs), other materials described herein, or combinations thereof.

The agents within these matrices can include any agent disclosed herein or combinations thereof, including radioactive materials; radiopaque materials; cytogenic agents; cytotoxic agents; cytostatic agents; thrombogenic agents, for example polyurethane, cellulose acetate polymer mixed with bismuth trioxide, and ethylene vinyl alcohol; lubricious, hydrophilic materials; phosphor cholene; anti-inflammatory agents, for example non-steroidal anti-inflammatories (NSAIDs) such as cyclooxygenase-1 (COX-1) inhibitors (e.g., acetylsalicylic acid, for example ASPIRIN® from Bayer AG, Leverkusen, Germany; ibuprofen, for example ADVIL® from Wyeth, Collegeville, PA; indomethacin; mefenamic acid), COX-2 inhibitors (e.g., VIOXX® from Merck & Co., Inc., Whitehouse Station, NJ; CELEBREX® from Pharmacia Corp., Peapack, NJ; COX-1 inhibitors); immunosuppressive agents, for example Sirolimus (RAPAMUNE®, from Wyeth, Collegeville, PA), or matrix metalloproteinase (MMP) inhibitors (e.g., tetracycline and tetracycline derivatives) that act early within the pathways of an inflammatory response. Examples of other agents are provided in Walton et al, Inhibition of Prostoglandin E2 Synthesis in Abdominal Aortic Aneurysms, Circulation, July 6, 1999, 48-54; Tambiah et al, Provocation of Experimental Aortic Inflammation Mediators and Chlamydia Pneumoniae, Brit. J. Surgery 88 (7), 935-940; Franklin et al, Uptake of Tetracycline by Aortic Aneurysm Wall and Its Effect on Inflammation and Proteolysis, Brit. J. Surgery 86 (6), 771-775; Xu et al, Sp1 Increases Expression of Cyclooxygenase-2 in Hypoxic Vascular Endothelium, J. Biological Chemistry 275 (32) 24583-24589; and Pyo et al, Targeted Gene Disruption of Matrix Metalloproteinase-9 (Gelatinase B) Suppresses Development of Experimental Abdominal Aortic Aneurysms, J. Clinical Investigation 105 (11), 1641-1649 which are all incorporated by reference in their entireties.

Any elements described herein as singular can be pluralized (i.e., anything described as "one" can be more than one). Any species element of a genus element can have the characteristics or elements of any other species element of that genus. The above-described configurations, elements or complete assemblies and methods and their elements for carrying out the invention, and variations of aspects of the invention can be combined and modified with each other in any combination.

The claims of the parent application are reproduced below on pages 17-18. These clauses define preferred embodiments. The applicant reserves the right to pursue protection for the combinations of features set out in these clauses, and/or for any other subject-matter contained in the parent application as filed, either in the present divisional application or in a further application divided from the present divisional application. The claims of the parent application are not the claims of this divisional application. The claims of the current divisional application are contained in a separate section on pages numbered 19-20 and headed "Claims".

### Preferred Embodiments

1. A device configured to treat a biological tunnel defect in a septum made of tissue, comprising: a resiliently flexible frame; a first anchor, a second anchor, and a third anchor, wherein the first anchor and the second anchor are positioned toward a first longitudinal end of the device, and wherein the third anchor is positioned toward a second longitudinal end of the device, and wherein the first anchor is located opposite the second anchor, and wherein the first anchor, second anchor, and third anchor are configured to attach to the tissue; a scaffold coupled to the frame, wherein the scaffold can be configured to substantially fill the tunnel defect; and wherein the scaffold is configured to encourage growth of the tissue onto the scaffold.
2. The device of clause 1, wherein the scaffold comprises a mesh.
3. The device of clause 1 or clause 2, wherein the scaffold comprises a metal.
4. The device of any one of the preceding clauses, wherein the scaffold comprises a shape-memory metal.
5. The device of any one of the preceding clauses, wherein the scaffold comprises Nitinol.
6. The device of any one of the preceding clauses, wherein the first anchor and the second anchor are resiliently flexible.
7. The device of any one of the preceding clauses, wherein the first anchor and the third anchor are configured to clamp the tissue between the first anchor and the third anchor.
8. The device of any one of the preceding clauses, further comprising a fourth anchor, wherein the second anchor and the fourth anchor are configured to clamp the tissue between the second anchor and the fourth anchor.
9. The device of any one of the preceding clauses, the first anchor can be a passive anchor.
10. The device of any one of clauses 1 to 8, the first anchor can be an active anchor.
11. The device of any one of the preceding clauses, wherein the scaffold comprises a fabric.
12. The device of any one of clauses 1 to 10, wherein the scaffold comprises a filament.
13. The device of any one of clauses 1 to 10, wherein the scaffold is non-woven.
14. The device of any one of clauses 1 to 10, wherein the scaffold comprises a film.
15. The device of any one of the preceding clauses, wherein the scaffold is configured to plug the tunnel defect.
16. The device of clause 1, wherein the device is biodegradable.
17. The device of any one of clauses 1 to 15, wherein the device is non-biodegradable.
18. The device of any one of the preceding clauses, wherein the device comprises a drug.
19. The device of clause 18, wherein the drug is a component of a coating.
20. The device of clause 1, comprising a non-made-material.
21. The device of clause 20, wherein the non-made-material comprises a bone chip.
22. The device of clause 1, comprising a polymer.
23. The device of clause 22, wherein the polymer comprises polylactic acid.
24. The device of clause 22, wherein the polymer comprises polyglycolic acid.
25. The device of clause 1, wherein the scaffold is non-porous.
26. The device of any one of the preceding clauses, wherein the device is configured to meet the contours of the tunnel defect.
27. The device of any one of clauses 1 to 25, wherein the device is configured to conform to the substantially exact shape of the tunnel defect.
28. The device of any one of the preceding clauses, wherein the scaffold comprises a laser cut film.
29. The device of any one of the preceding clauses, wherein the scaffold comprises a sheet.
30. The device of any one of clauses 1 to 28, wherein the scaffold comprises a tube.

## Claims

1. A device (10) configured to treat a biological tunnel defect in a septum made of tissue, comprising:
a flat, resiliently flexible frame (12) comprising first and second arms (20; 22) disposed in a first plane;
an internal element (14) attached to the frame;
a first anchor (26; 24) and a second anchor (26; 24) extending from a first longitudinal end of the frame and being configured to engage a first side of the biological tunnel defect;
a third anchor (24; 26) and a fourth anchor (24; 26) extending from a second longitudinal end of the frame being configured to engage a second side of the biological tunnel defect; and
wherein the frame is configured to expand outward in the first plane within the biological tunnel defect to distend the tissue outward, and wherein the first anchor and the second anchor are configured to pull toward the third anchor and the fourth anchor along the first plane.

2. The device of claim 1, wherein the first (26; 24), second (26; 24), third (24; 26) and fourth anchors (24; 26) each comprise an arcuate length.

3. The device of claim 1, wherein the first anchor (26; 24) is longer than the third anchor (26; 24).

4. The device of claim 1, wherein the internal element (14) is configured to contact the tissue of the biological tunnel defect.

5. The device of claim 1, wherein the first (26; 24) and second anchors (26; 24) are configured to move away from each other along the first plane when the frame expands.

6. The device of claim 5, wherein the third (24; 26) and fourth anchors (24; 26) are configured to move away from each other along the first plane when the frame expands.

7. The device of claim 1, wherein the frame (12) is configured to compress inward in the first plane within a catheter for delivery.

8. The device of claim 7, wherein the first (26; 24) and second anchors (26; 24) are configured to move towards each other along the first plane when the frame compresses.

9. The device of claim 8, wherein the third (24; 26) and fourth anchors (24; 26) are configured to towards each other along the first plane when the frame compresses.

10. The device of claim 1, wherein the internal element (14) comprises a mesh.

11. The device of claim 1, wherein the internal element (14) comprises a shape-memory metal.

12. The device of claim 1, wherein the internal element (14) comprises a filament.

13. The device of claim 1, wherein the internal element (14) comprises a thrombosis promoting material.

14. The device of claim 1, wherein the internal element (14) comprises a tissue in-growth promoting material.

15. The device of claim 1, wherein the internal element (14) is configured to plug the tunnel defect.
